# EUROPÄISCHE PATENTSCHRIFT

(11) **EP 1 140 826 B1**
(45) Veröffentlichungstag und Bekanntmachung des Hinweises auf die Patenterteilung: **06.05.2004**
(21) Anmeldenummer: 99966978.1
(22) Anmeldetag: 21.12.1999
(51) Int. Cl.: C07D 209/42, C07D 209/08, C07D 209/34, C07C 223/04, C07C 217/52, C07C 205/33, C07C 205/28, C07C 205/29

(54) **VERFAHREN ZUR HERSTELLUNG VON (2S,4R,9S)-OCTAHYDRO-1H-INDOL-2-CARBONSÄURE UND ZWISCHENPRODUKTE DAFÜR**
METHOD FOR PRODUCING (2S,4R,9S)-OCTAHYDRO-1H-INDOLE-2-CARBOXYLIC ACID AND INTERMEDIATE PRODUCTS THEREFOR
PROCEDE DE PREPARATION D'ACIDE (2S,4R,9S)-OCTAHYDRO-1H-INDOL-2-CARBOXYLIQUE ET PRODUITS INTERMEDIAIRES APPROPRIES

(30) Priorität: 07.01.1999 DE 19900205
(43) Veröffentlichungstag der Anmeldung: 10.10.2001
(73) Patentinhaber: Abbott GmbH & Co. KG, 65205 Wiesbaden (DE)
(72) Erfinder: EBEL, Klaus, D-68623 Lampertheim (DE); OHLBACH, Frank, D-69221 Dossenheim (DE); NÜBLING, Christoph, D-67454 Ha loch (DE)
(74) Vertreter: Grünecker, Kinkeldey, Stockmair & Schwanhäusser Anwaltssozietät
(86) Internationale Anmeldenummer: PCT/EP1999/010159
(87) Internationale Veröffentlichungsnummer: WO 2000/040555

(56) Entgegenhaltungen:
- EP-A- 0 084 164
- EP-A- 0 132 580
- BRION F ET AL: "STEREOSELECTIVE SYNTHESIS OF A TRANS-OCTAHYDROINDOLE DERIVATIVE, PRECURSOR OF TRANDOLAPRIL (RU 44 570), AN INHIBITOR OF ANGIOTENSIN CONVERTING ENZYME" TETRAHEDRON LETTERS., Bd. 33, Nr. 34, 1992, Seiten 4889-4892, XP000885877 ELSEVIER SCIENCE PUBLISHERS, AMSTERDAM., NL ISSN: 0040-4039
- YANOVSKAYA L A ET AL: BULLETIN OF THE RUSSIAN ACADEMY OF SCIENCES. DIVISION OF CHEMICAL SCIENCE.,1962, Seiten 623-629, XP000886172 PLENUM PUBLISHING CO, NEW YORK, NY., US

## Beschreibung

Die vorliegende Erfindung betrifft ein Verfahren zur Herstellung von (2S,4R,9S)-Octahydro-1H-indol-2-carbonsäure und Zwischenprodukte, die bei dieser Herstellung verwendet werden.
(2S,4R,9S)-Octahydro-1H-indol-2-carbonsäure besitzt die Formel 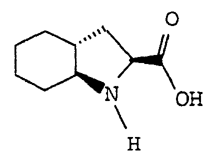 (2S,4R,9S)-Octahydro-1H-indol-2-carbonsäure ist ein wichtiges Zwischenprodukt zur Herstellung von Angiotensinase-Inhibitoren (DE 3.322.530, EP 267.098). Insbesondere ist es ein Schlüsselprodukt bei der Herstellung von Trandolapril (EP 84.164).

Die bislang bekannten Herstellwege für (2S,4R,9S)-Octahydro-1H-indol-2-carbonsäure sind sehr aufwendig (EP 267 098, DE 3322530). Es wurde nun ein wesentlich einfacheres und kostengünstigeres Verfahren zur Herstellung dieser Substanz gefunden.

Gegenstand der Erfindung ist ein Verfahren zur Herstellung von (2S,4R,9S)-Octahydro-1H-indol-2-carbonsäure, welches darin besteht, daß man
a. eine Verbindung der Formel I

   (R¹O)₂CH-CH₂-CH(OR²)₂ I

   worin R¹ und R² gleich oder verschieden sind und C₁₋₄-Alkylgruppen darstellen, mit Wasser in Gegenwart eines sauren Katalysators umsetzt,
b. die so erhaltenen 3,3-Dialkoxypropionaldehyde der Formel II

   (R¹O)₂CH-CH₂-CHO II

   einer Henry-Reaktion mit Nitromethan unterwirft,
c. das so erhaltene 4,4-Dialkoxy-1-nitro-2-butanol der Formel III

   (R¹O)₂CH-CH₂-CHOH-CH₂-NO₂ III

   dehydratisiert,
d. das so erhaltene Nitroolefin IV mit Hilfe einer Diels-Alder-Reaktion in das entsprechende trans-4-(2,2-Dialkoxyethyl)-5-nitro-1-cyclohexen V 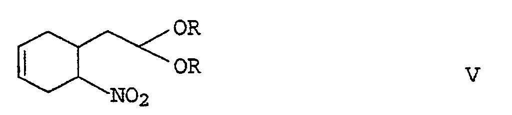 überführt,
e. die so erhaltene Substanz V zum entsprechenden trans-4-(2,2-Dialkoxyethyl)-5-amino-1-cyclohexan VI 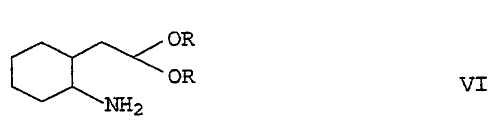 hydriert,
f. die Verbindung VI einer Racematspaltung unterwirft und das (1S,2R)-1-Amino-2-(2,2-dialkoxyethyl)-cyclohexan VII 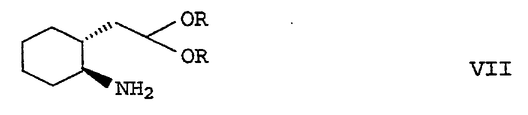 in enantiomerenreiner Form gewinnt,
g. die so erhaltene Verbindung VII zum entsprechenden Aldehyd VIII 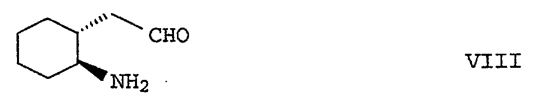 hydrolysiert,
h. den so erhaltenen Aldehyd durch Umsetzung mit Cyanid-Ionen in das entsprechende Nitril IX 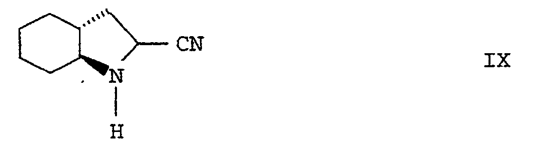 überführt und
i. dieses Nitril zur (2S,4R,9S)-Octahydro-1H-indol-2-carbonsäure verseift.

Die Hydrolyse der 1,1,3,3-Tetraalkoxyalkane (I) zu den entsprechenden 3,3-Dialkoxypropionaldehyden (= Malondialdehyd-Monoacetalen) (II) wird durch Umsetzung des Edukts mit Wasser und einem sauren Katalysator durchgeführt. Als Katalysatoren sind im Prinzip alle für die Hydrolyse von Acetalen bekannten Katalysatoren einsetzbar. Insbesondere geeignet sind starke Protonensäuren oder stark saure Ionenaustauscher, wie z.B. Schwefelsäure, Salzsäure, Phosphorsäure, Toluolsulfonsäure, Nafion, Ionenaustauscher mit Sulfonsäure-Gruppen etc.

Die Stufe b, die Henry-Reaktion des Malondialdehyd-Monoacetals mit Nitromethan zum 4,4-Dialkoxy-1-nitro-2-butanol (III) wird unter den für solche Additionen üblichen Bedingungen durchgeführt (M. Shvekhgeimer, Russ. Chem Rev. 67, 35-68 (1998)). Als Katalysatoren kommen alle für solche Reaktionen beschriebenen Katalysatoren in Betracht wie beispielsweise stickstoffhaltige Basen, wie aliphatische Amine oder Guanidine, basische Ionenaustauscher, Kaliumfluorid, Kaliumfluorid auf Aluminiumoxid, Alkali- oder Erdalkali-Hydroxide und Alkali- oder Erdalkali-Alkoholate, wie Natriummethylat geeignet. Die Stufe b wird in der Regel unter basischen Bedingungen durchgeführt. Als Basen eignen sich insbesondere Amine, bevorzugt tertiäre Amine, wie beispielsweise Trimethylamin, Triethylamin, Tetramethylethylendiamin, Tetramethyl-1,3-propandiamin, DBN und DABCO. Das Malondialdehyd-Monoacetal kann als Rohprodukt, wie es bei der Hydrolyse anfällt, problemlos ohne eine spezielle Reinigung weiterverarbeitet werden.

In der Stufe c wird das 4,4-Dialkoxy-1-nitro-2-butanol (III) zum 4,4-Dialkoxy-1-nitro-2-buten (IV) dehydratisiert. Für die Dehydratisierung können alle für die Dehydratisierung von beta-Nitroalkoholen bekannten Methoden verwendet werden. Als solche seien genannt: 1. Die direkte Dehydratisierung mit Aluminiumoxid (J.Org.Chem. 57, 2160-2162 (1992)), 2. die Dehydratisierung mit Methansulfonsäurechlorid und Triethylamin (J.Org.Chem. 40, 2138-2139 (1975)) und 3. die Dehydratisierung mittels Phthalsäureanhydrid (Org. Synth. 60, 101 (1981).

Die Dehydratisierung c kann auch durch Acylierung des Alkohols mit einem Säureanhydrid und anschließende Abspaltung von Säure mit Basen wie Aylkalicarbonaten oder Alkalihydrogencarbonaten oder stickstoffhaltigen Basen oder Aluminiumoxid vorgenommen werden, vgl. J.Am.Chem.Soc. 76, 2716 (1954), J. Am.Chem.Soc. 69, 1048 (1947), Synthesis 1983, 920, Liebigs Ann.Chem. 1994, 1235 und Tetrahydron Lett. 35, 5731 (1994). Weitere Dehydratisierungsmöglichkeiten sind die mit Dicyclohexylcarbodiimid unter Kupfer-Katalyse (Synthesis 1982, 1017) und die mit Triphenylphosphin/Tetrachlorkohlenstoff/-Triethylamin (Synthesis 1994, 685).

Als besonders günstig hat sich für die Dehydratisierung c erwiesen, den Nitroalkohol mit Acetanhydrid zu acylieren und anschließend Essigsäure abzuspalten. Dabei wird die Essigsäure zum Teil bereits während der Acylierung abgespalten. Die vollständige Abspaltung kann entweder thermisch bei Temperaturen von 120 bis 500°C oder mit Basen, wie Alkali- oder Erdalkali-carbonaten, - hydrocarbonaten oder -hydroxiden der aliphatischen Aminen erfolgen. Für die Acylierung werden vorteilhaft Katalysatoren (beispielsweise 4-(Dimethylamino)pyridin) eingesetzt. Bei der Dehydratisierung entsteht überwiegend das thermodynamisch stabilere trans-Produkt.

In der Stufe d wird das Nitroolefin mit Butadien in einer Diels-Alder-Reaktion umgesetzt. Bei rein thermischer Durchführung ohne Katalysator wird die Diels-Alder-Reaktion bei Temperaturen zwischen 50 und 200°C, bevorzugt zwischen 90 und 120°C, in aromatischen Kohlenwasserstoffen wie Benzol, Toluol oder Xylol durchgeführt. Durch den Einsatz von Katalysatoren kann die Reaktionstemperatur abgesenkt werden. Aus dem trans-Nitroolefin entsteht dabei trans-4-(2,2-Dialkoxyethyl)-5-nitro-1-cyclohexen.

In der Stufe e werden die Doppelbindung und die Nitrogruppe hydriert. Die Hydrierung kann ein- oder zweistufig durchgeführt werden. Als Katalysatoren sind alle Hydrierkatalysatoren, vorzugsweise Raney-Nickel oder Pd- oder Pt-Katalysatoren wie z.B. Pd/C oder Pt/C geeignet. Die Hydrierung wird bei 20 bis 150°C durchgeführt. Als Lösungsmittel eignen sich: Alkohole wie Methanol und Ethanol oder Essigsäure.

Die Herstellung von enantiomerenreinem (1S,2R)-1-Amino-2-(2,2-dialkoxyethyl)-cyclohexan (Stufe f) gelingt durch Racemattrennung in üblicher Weise. Als besonders geeignet hat sich die enzymatische Racematspaltung erwiesen. Hierbei wird die racemische Mischung von (1S,2R)- und (1R,2S)-1-Amino-2-(2,2-dialkoxyethyl)-cyclohexan mit Acylierungsmitteln wie Alkoxyessigsäureisopropylester in Gegenwart von Hydrolasen, insbesondere Lipasen umgesetzt. Dabei wird das (1R,2S)-Enantiomer selektiv acyliert, während das gewünschte (1S,2R)-Enantiomer nicht reagiert und aus dem Reaktionsgemisch destillativ oder chromatisch abgetrennt werden kann. Als Lipase eignet sich besonders Novozym 435. Die Spaltung erfolgt zweckmäßig bei 20 bis 40°C.

Die Hydrolyse des Acetals (Stufe g) zum Aldehyd erfolgt zweckmäßig durch Kochen des Acetals in wässriger Säure wie Salzsäure, Schwefelsäure oder Phosphorsäure.

Die Überführung des Aldehyds in das Nitril (Stufe h) gelingt besonders gut durch Umsetzung mit Natriumcyanid unter alkalischen Bedingungen bei PH > 9, wobei das Nitril direkt zum Natriumsalz der (2S,4R,9S)-Octahydro-1H-indol-2-carbonsäure verseift wird (Stufe i). Die Säure wird dann durch Ansäuern der Reaktionslösung erhalten.

Gegenstand der Erfindung sind auch die neuen Verbindungen, über die die oben beschriebene Synthese läuft. Es sind dies:
2. 4,4-Dialkoxy-1-nitro-2-butene der Formel IV

   (R¹O)₂CH-CH=CH-CH₂-NO₂ IV,
3. trans-4-(2,2-Dialkoxyethyl)-5-nitro-1-cyclohexene der Formel V 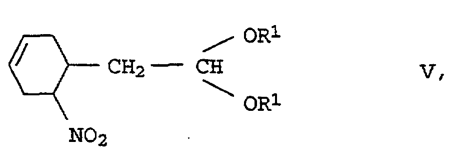
4. trans-4-(2,2-Dialkoxyethyl)-5-amino-1-cyclohexane der Formel VI 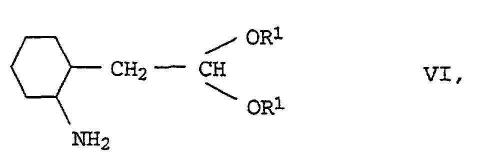
5. (1S,2R)-1-Amino-2-(2,2-dialkoxyethyl)-cyclohexane der Formel VII 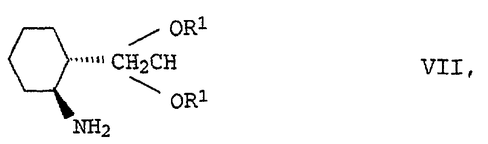
6. der Aldehyd der Formel VIII 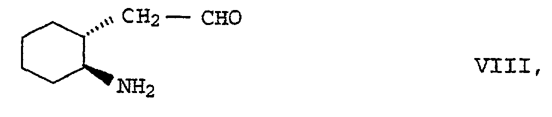
sowie dessen Säureadditionssalze.

Das neue Verfahren zur Herstellung von (2S,4R,9S)-Octahydro-1H-indol-2-carbonsäure liefert die Substanz in einer deutlich besseren Ausbeute als die bislang bekannten Verfahren zu ihrer Herstellung. Weiter ist die Herstellung der Substanz dadurch stark vereinfacht, daß die Synthese über weniger Stufen führt. Schließlich sind die als Ausgangsmaterialien verwendeten 1,1,3,3-Tetraalkoxyalkane in technischem Maßstab kostengünstig herstellbare Ausgangsmaterialien.

Die neuen Zwischenprodukte stellen die Schlüsselsubstanzen für die Synthese dar.

### Beispiele

### Beispiel 1

### Herstellung von 3,3-Dimethoxypropanal mit saurem Ionentauscher als Katalysator

Man kochte eine Mischung aus 2756 g (16 Mol) 95%igem 1,1,3,3-Tetramethoxypropan, 292 g (16 Mol) Wasser, 256 g (8 Mol) Methanol und 20 g stark saurem Ionentauscher (BayKat. K 2431) in einem 6 l Rührreaktor aus Edelstahl 1,5 h unter Rückfluß. Anschließend wurde auf 50°C abgekühlt und der Ionentauscher abfiltriert. Das Filtrat wurde auf pH 2,5 eingestellt und über eine Kolonne mit 20 theoretischen Böden destilliert. Man erhielt folgende Fraktionen:

| Frakt. Nr. | Druck [mbar] | Temp. [°C] | Menge [g] | Zusammensetzung |
|---|---|---|---|---|
| 1 | 400-30 | 42-22 | 1200 | 87 % Methanol, 9 % Wasser |
| 2 | 20 | 30-51 | 84 | 56,2 % Dimethoxypropionaldehyd |
| 3 | 20 | 51-57 | 575 | 98,9 % Dimethoxypropionaldehyd |
| 4 | 20 | 57-69 | 349 | 68,6 % Dimethoxypropionaldehyd |
| | | | | 31,0 % Tetramethoxypropan |
| 5 | 20 | 69-71 | 816 | 98,3 % Tetramethoxypropan |
| Rückstand | | | 260 | |

Fraktion 3 wurde für die nächste Stufe eingesetzt. Die Fraktion 4 wurde redestilliert und die bei 51 - 57°C überdestillierende Fraktion (239 g) zur Fraktion 3 hinzugefügt.

Von den 2756 g (16 mol) eingesetztem 95%igem Tetramethoxypropan wurden 925 g (5,6 mol) zurückgewonnen. Daraus folgt ein Verbrauch von 1831 g (10,4 mol) Tetramethoxypropan, das heißt, daß 65 % des eingesetzten Tetramethoxypropans umgesetzt worden waren. Die Fraktionen 3 und 4 enthielten zusammen 575 g + 237 g (= 68,6 % von 349 g) = 812 g Dimethoxypropionaldehyd. Das entspricht einer Ausbeute von 66 % bezogen auf umgesetztes Tetramethoxypropan.

### Beispiel 2

### 4,4-Dimethoxy-1-nitro-2-butanol

Eine Mischung aus 122 g (2 mol) Nitromethan und 122 g Methanol wurde vorgelegt und unter Kühlung bei 20 - 30°C 120,4 g (1 mol) 98%iger 3,3-Dimethoxypropionaldehyd zugetropft. Anschließend ließ man 52 g (0,25 mol) 45%ige wäßrige Trimethylamin-Lösung zulaufen und rührte noch 2 h bei 50°C nach. Dann wurden die niedrig siedenden Bestandteile bei einer Sumpftemperatur von maximal 60°C im Vakuum (zum Ende des Abdestillierens bis 30 mbar) abdestilliert. Man erhielt 181,5 g eines Öls, welches nach GC-Analyse 157,7 g (0,98 mol) 4,4-Dimethoxy-1-nitro-2-butanol (Rohausbeute: 98 %) enthielt. Das Rohprodukt wurde ohne weitere Reinigung in die nächste Stufe eingesetzt. Zur Bestimmung der analytischen Daten wurde nach Filtration über Aluminiumoxid am Kugelrohr destilliert:
¹H-NMR (CDCl₃) nach Kugelrohrdestillation: δ(ppm) = 1,82 (t, 2H, C-CH₂-C), 3,35 (s, 6H, O-CH₃), 3,84 (s, 1 H, OH), 4,4 - 4,6 (m, 3 H, CH-O und CH₂-NO₂), 4,62 (t, 1 H, CH(OMe)₂)

### Beispiel 3

### trans-4,4-Dimethoxy-1-nitro-1-buten

Das Rohprodukt aus der vorhergehenden Stufe (181 g) wurde in 360 ml Essigester gelöst und 1,2 g (0,01 Mol) 4-Dimethylaminopyridin und 100,8 g (1,2 mol) festes Natriumhydrogencarbonat zugegeben. Dann tropfte man 153 g (1,5 mol) Acetanhydrid zu, wobei die Temperatur auf 35°C anstieg und rührte noch 2 h bei 50°C nach. Anschließend wurde die Reaktionsmischung mit 100 ml Wasser versetzt, wobei Kohlendioxid freigesetzt wurde. Nach Abtrennung der Wasserphase wurde die organische Phase noch 2 mal mit je 100 ml gesättigter Natriumhydrogencarbonat-Lösung gewaschen und die organische Phase am Rotationsverdampfer eingeengt. Der Rückstand wurde bei 4 mbar und 130°C Wandtemperatur über einem Sambay-Verdampfer destilliert. Man erhielt 18 g Rückstand und 151 g orangerot gefärbtes Destillat, welches aus 68 % 4,4-Dimethoxy-1-nitro-1-buten, 10 % 2-Acetoxy-4,4-dimethoxy-1-nitro-1-butan, 1,5 % 4,4-Dimethoxy-1-nitro-2-butanol und 17 % Essigsäure bestand. Das Destillat wurde in 300 ml Essigester gelöst und 2 mal mit je 100 ml einer gesättigten Natriumhydrogencarbonat-Lösung gewaschen. Dann wurde die organische Phase abgetrennt, der Essigester am Rotationsverdampfer entfernt und der Rückstand nochmals destilliert. Man erhielt 128 g 4,4-Dimethoxy-1-nitro-1-buten mit einer Reinheit nach GC von 80 %. Das entspricht einer Ausbeute von 60 % ausgehend von 3,3-Dimethoxypropionaldehyd.

Dieses Produkt wird ohne weitere Reinigung in die nächste Stufe eingesetzt.

Durch die Destillation wurde eine Probe mit 95 % GC-Reinheit erhalten:
¹H-NMR(CDCl₃): δ = 2,60 (t, 2 H, C-CH₂-C), 3,35 (s, 6 H, O-CH₃), 4,52 (t, 1 H, CH(OMe)₂), 7,05 - 7,3 (m, 2 H, Olefin-H). Durch Feinanalyse der olefinischen Protonen wurde ein trans:cis-Verhältnis von 14,2:1 ermittelt.

### Beispiel 4

### trans-4-(2,2-Dimethoxyethyl)-5-nitro-1-cyclohexen

Man legte 214 g (1,0 mol) 75%iges 4,4-Dimethoxy-1-nitro-1-buten und 0,1 g Phenothiazin in einem Autoklaven bei Raumtemperatur in 850 ml Toluol vor und preßte dann 216 g (4,0 mol) Butadien auf. Anschließend wurde unter Eigendruck (etwa 6 bar) 30 h bei 100°C gerührt. Dann wurde abgekühlt und entspannt. Schließlich destillierte man das Toluol und nicht umgesetztes Butadien ab. Man erhielt 270 g eines öligen Rückstandes, welcher nach GC-Analyse etwa 39 % Reaktionsprodukt und noch etwa 10 % Edukt enthielt. Das Rohprodukt wurde direkt ohne weitere Reinigung in die nachfolgende Hydrierung eingesetzt.

Durch Kugelrohrdestillation wurde eine Probe mit 95 % GC-Reinheit erhalten:
¹H-NMR(CDCl₃) : δ(ppm) = 1,5 (m, 1H), 1,7 (m, 1H), 1,9 (m, 1H), 2,35 - 2,75 (m, 4H), 3,30 (2s, 6 H, O-CH₃), 4,5 (m, 2 H, CH(OMe)₂ und CHNO₂), 5, 6 - 5,7 (m, 2 H, Olefin-H).

### Beispiel 5

### trans-1-Amino-2-(2,2-dimethoxyethyl)cyclohexan

270 g des Rückstands aus der vorhergehenden Stufe wurden in 540 ml Methanol aufgenommen und über Natriumsulfat filtriert. Dann wurde mit 21 g 10 % Pd/C als Katalysator 10 h bei 150 bar Wasserstoffdruck und 100°C hydriert. Anschließend filtrierte man den Katalysator ab, wusch mit 100 ml Methanol nach und engte das Filtrat ein. Der Rückstand wurde im Vakuum (6 mbar, 130°C) über einen Sambay-Verdampfer destilliert. Man erhielt 120 g Destillat mit einem Gehalt von etwa 57 % trans-1-Amino-2-(2,2-dimethoxyethyl)cyclohexan. Die Ausbeute über beide Stufen ausgehend von 4,4-Dimethoxy-1-nitro-1-buten betrug 36 %.

Das Produkt konnte durch Zugabe von Essigsäure als Acetat kristallisiert werden. Dazu wurde das rohe Amin in Diethylether gelöst, mit Essigsäure versetzt (auf 10 g Amin 300 ml Ether und 32 g Essigsäure) und das ausgefallene Acetat abgesaugt. Anschließend wurde das Acetat aus Essigester umkristallisiert. Man erhielt 1-Amino-2-(2,2-dimethoxyethyl)cyclohexan-acetat vom Schmp. 92 - 94°C. Zur Bestimmung der analytischen Daten wurde die freie Base aus dem Acetat durch Zugabe von Natronlauge freigesetzt, mit Essigester extrahiert, der Extrakt eingedampft und am Kugelrohr destilliert.
¹H-NMRR (CDCl₃): δ(ppm) = 0, 9 - 1, 4 (m, 8H), 1, 6 - 1,9 (m, 4H), 1,98 - 2,1 (m, 1H)J, 2,1 - 2,2 (m, 1H), 3,36 (2s, 6 H, O-CH₃), 4,50 (m, 1 H, CH(OMe)₂)

### Beispiel 6

### (1S.2R)-1-Amino-2-(2,2-dimethoxyethyl)cyclohexan

Eine trockne Lösung von 2,1 g (11,2 mMol) trans-1-Amino-2-(2,2-dimethoxyethyl)-cyclohexan und 1,38 g (10,4 mMol) Methoxyessigsäureisopropylester in 20 ml MTBE wurde mit 200 mg der immobilisierten Lipase Novozym® 435 versetzt und 25 h bei Raumtemperatur geschüttelt. Danach lag der Umsatz bei 49,5 %. Das (1R,2S)-Enantiomere wurde zum Methoxyacetamid acyliert, während das (1S,2R)-Enantiomere nicht acyliert wurde. Nach Abfiltrieren des Enzyms, Abdampfen des Lösungsmittels am Rotationsverdampfer und säulenchromatographischer Reinigung (Kieselgel, Elution anfangs mit MTBE anschließend mit MTBE/Methanol) wurden 970 mg reines (1S,2R)-Amino-2-(2,2-dimethoxyethyl)cyclohexan (46 % Ausbeute) erhalten.

### Beispiel 7

### (2S,4R,9S)-Octahydroindol-2-carbonsäure

2,25 g (0,01 mol) (1S,2R)-1-Amino-2-(2,2-dimethoxyethyl)cyclohexan wurden in 50 ml mit 1 N Salzsäure 30 min am Rückfluß gekocht. Dann wurde die Lösung mit Natronlauge auf pH 6 - 8 eingestellt, anschließend 0,7 g (0,015 mol) Natriumcyanid zugegeben und der pH-Wert mit weiterer Natronlauge auf 11 eingestellt. Anschließend kochte man noch 1 h am Rückfluß. Dann wurde abgekühlt, mit Salzsäure auf pH 6 eingestellt, das Produkt mit Essigester kontinuierlich extrahiert und der Extrakt eingeengt. Man erhielt 1,7 g eines nach DC einheitlichen öligen Rückstandes. Dieser Rückstand wurde in Methylenchlorid gelöst und das Produkt durch Einleiten von Chlorwasserstoff als Hydrochlorid isoliert. Das Produkt entspricht dem nach EP 267 098 Example 1, Stade C hergestellten Hydrochlorid.

## Patentansprüche

1. Verfahren zur Herstellung von (2S,4R,9S)-Octahydro-1H-indol-2-carbonsäure, **dadurch gekennzeichnet, daß** man
a. eine Verbindung der Formel I
(R¹O)₂CH-CH₂-CH(OR²)₂ I
worin R¹ und R² gleich oder verschieden sind und C₁₋₄-Alkylgruppen darstellen, mit Wasser in Gegenwart eines sauren Katalysators umsetzt,
b. die so erhaltenen 3,3-Dialkoxypropionaldehyde der Formel II
(R¹O)₂CH-CH₂-CHO II
einer Henry-Reaktion mit Nitromethan unterwirft,
c. das so erhaltene 4,4-Dialkoxy-1-nitro-2-butanol der Formel III
(R¹O)₂CH-CH₂-CHOH-CH₂-NO₂ III
dehydratisiert,
d. das so erhaltene Nitroolefin IV mit Hilfe einer Diels-Alder-Reaktion in das entsprechende trans-4-(2,2-Dialkoxyethyl)-5-nitro-1-cyclohexen V überführt,
e. die so erhaltene Substanz V zum entsprechenden trans-4-(2,2-Dialkoxyethyl)-5-amino-1-cyclohexan VI hydriert,
f. die Verbindung VI einer Racematspaltung unterwirft und das (1S,2R)-1-Amino-2-(2,2-dialkoxyethyl)-cyclohexan VII in enantiomerenreiner Form gewinnt,
g. die so erhaltene Verbindung VII zum entsprechenden Aldehyd VIII hydrolysiert,
h. den so erhaltenen Aldehyd durch Umsetzung mit Cyanid-Ionen in das entsprechende Nitril IX 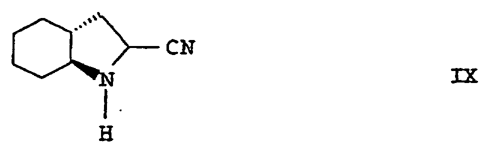 überführt und
i. dieses Nitril zur (2S,4R,9S)-Octahydro-1H-indol-2-carbon säure verseift.

2. 4,4-Dialkoxy-1-nitro-2-butene der Formel IV
(R¹O)₂CH-CH=CH-CH₂-NO₂ IV

3. trans-4-(2,2-Dialkoxyethyl)-5-nitro-1-cyclohexene der Formel V

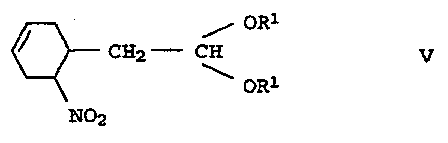

4. trans-4-(2,2-Dialkoxyethyl)-5-amino-1-cyclohexane der Formel VI

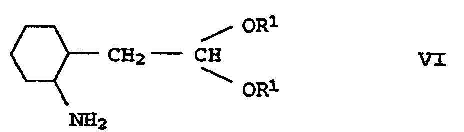

5. (1S,2R)-1-Amino-2-(2,2-dialkoxyethyl)-cyclohexane der Formel VII

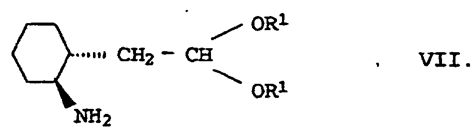

6. Aldehyd der Formel VIII 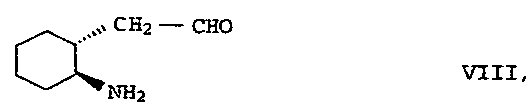 sowie dessen Säureadditionssalze.

## Claims

1. Process for the preparation of (2S,4R,9S)-octahydro-1H-indole-2-carboxylic acid, **characterised in that**
a. a compound of the formula I
(R¹O)₂CH-CH₂-CH(OR²)₂ I,
wherein R¹ and R² are the same or different and represent C₁₋₄ alkyl groups, is reacted with water in the presence of an acidic catalyst,
b. the 3,3-dialkoxypropionaldehydes of formula II thus obtained
(R¹O)₂CH-CH₂-CHO II
are subjected to a Henry reaction with nitromethane,
c. the 4,4-dialkoxy-1-nitro-2-butanol of formula III thus obtained
(R¹O)₂CH-CH₂-CHOH-CH₂-NO₂ III
is dehydrated,
d. the nitroolefin IV thus obtained is converted into the corresponding trans-4-(2,2-dialkoxyethyl)-5-nitro-1-cyclohexene V with the aid of a Diels-Alder reaction,
e. the substance V thus obtained is hydrogenated into the corresponding trans-4-(2,2-dialkoxyethyl)-5-amino-1-cyclohexane VI,
f. the compound VI is subjected to a resolution of racemates and the (1S,2R)-1-amino-2-(2,2-dialkoxyethyl)cyclohexane VII is obtained in enantiomerically pure form,
g. the compound VII thus obtained is hydrolysed to the corresponding aldehyde VIII,
h. the aldehyde thus obtained is converted by reaction with cyanide ions into the corresponding nitrile IX 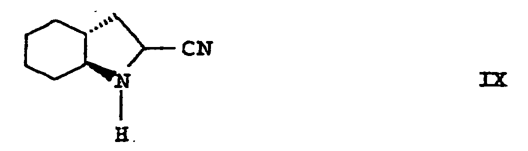 and
i. this nitrile is saponified to the (2S,4R,9S)-octahydro-1H-indole-2-carboxylic acid.

2. 4,4-Dialkoxy-1-nitro-2-butenes of formula IV
(R¹O)₂CH-CH=CH-CH₂-NO₂ IV.

3. trans-4-(2,2-Dialkoxyethyl)-5-nitro-1-cyclohexenes of formula V

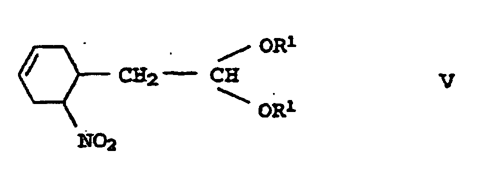

4. *trans*-4-(2,2-Dialkoxyethyl)-5-amino-1-cyclohexanes of formula VI

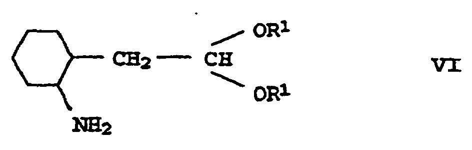

5. (1S,2R)-1-Amino-2-(2,2-dialkoxyethyl)cyclohexanes of formula VII

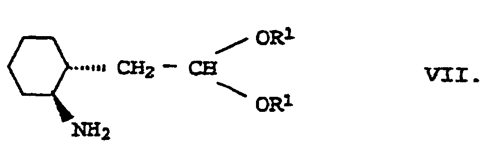

6. Aldehyde of formula VIII 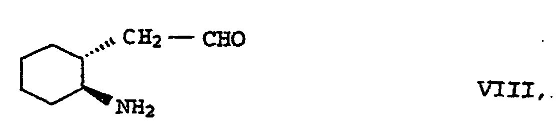 and its acid addition salts.

## Revendications

1. Procédé pour la préparation d'acide (2S,4R,9S)-octahydro-1H-indol-2-carboxylique, **caractérisé en ce que**
a. on fait réagir un composé de la formule I
(R¹O)₂CH-CH₂-CH(OR²)₂ I
où R¹ et R² sont identiques ou différents et représentent des groupes alkyle en C₁₋₄ avec de l'eau en présence d'un catalyseur acide,
b. on soumet le 3,3-dialcoxypropionaldéhyde de la formule II ainsi obtenu
(R¹O)₂CH-CH₂-CHO II
à une réaction de Henry avec du nitrométhane,
c. on déshydrate le 4,4-dialcoxy-1-nitro-2-butanol de la formule III ainsi obtenu
(R¹O)₂CH-CH₂-CHOH-CH₂-NO₂ III
d. on transforme la nitrooléfine IV ainsi obtenue à l'aide d'une réaction de Diels-Alder en le trans-4-(2,2-dialcoxyéthyl)-5-nitro-1-cyclohexène V correspondant,
e. on hydrogène la substance V ainsi obtenue en le trans-4-(2,2-dialcoxyéthyl)-5-amino-1-cyclohexane VI correspondant,
f. on soumet le composé VI à une dissociation du racémate et on récupère Je (1S,2R)-1-amino-2-(2,2-dialcoxyéthyl)-cyclohexane VII dans une forme pure d'énantiomère,
g. on hydrolyse le composé VII ainsi obtenu en l'aldhéhyde VIII correspondant,
h. on transforme l'aldéhyde ainsi obtenu par réaction avec des ions cyanure en le nitrile IX correspondant 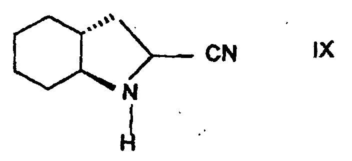 et
i. on saponifie ce nitrile en l'acide (2S,4R,9S)-octahydro-1H-indol-2-carboxylique.

2. 4,4-dialcoxy-1-nitro-2-butène de la formule IV
(R¹O)₂CH-CH=CH-CH₂-NO₂ IV

3. Trans-4-(2,2-dialcoxyéthyl)-5-nitro-1-cyclohexène de la formule V

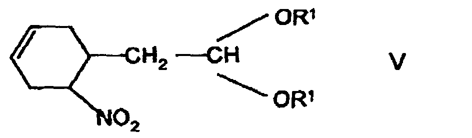

4. Trans-4-(2,2-dialcoxyéthyl)-5-amino-1-cyclohexane de la formule VI

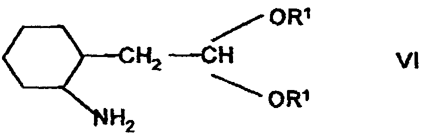

5. (1S,2R)-1-amino-2-(2,2-dialcoxyéthyl)-cyclohexane de la formule VII

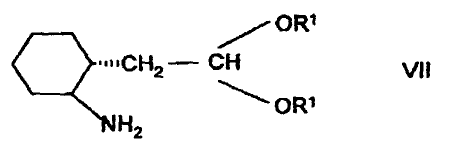

6. Aldéhyde de la formule VIII 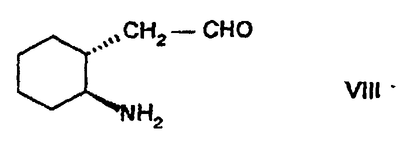 ainsi que ses sels d'addition d'acides.
